(19) Europäisches Patentamt
European Patent Office
Office européen des brevets

(11) **EP 3 968 118 B1**

(12) **EUROPÄISCHE PATENTSCHRIFT**

(45) Veröffentlichungstag und Bekanntmachung des Hinweises auf die Patenterteilung:
**09.11.2022  Patentblatt 2022/45**

(21) Anmeldenummer: **20196212.3**

(22) Anmeldetag: **15.09.2020**

(51) Internationale Patentklassifikation (IPC):
**G01N 9/36** (2006.01)   **G01F 1/74** (2006.01)
**G01F 1/86** (2006.01)   **G01F 13/00** (2006.01)
**G05D 11/13** (2006.01)   **G01N 33/18** (2006.01)

(52) Gemeinsame Patentklassifikation (CPC):
**G05D 11/137; G05D 11/131;** G01F 1/74;
G01F 1/86; G01F 13/00; G01N 9/36; G01N 33/18

(54) **VERFAHREN ZUR BESTIMMUNG EINES MITTLEREN GEHALTS EINER KOMPONENTE IN EINEM FLIESSENDEN STOFFGEMISCH**

METHOD FOR DETERMINING A MEAN CONTENT OF A COMPONENT IN A FLUID MIXTURE

PROCÉDÉ DE DÉTERMINATION DE LA TENEUR MOYENNE D'UN COMPOSANT DANS UN MÉLANGE FLUIDE DE MATIÈRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.03.2022  Patentblatt 2022/11**

(73) Patentinhaber: **Flexim Flexible Industriemesstechnik GmbH 12681 Berlin (DE)**

(72) Erfinder:
• **Hering, Jörg**
**15370 Fredersdorf-Vogelsdorf (DE)**
• **Hollatz, Ivo**
**16341 Panketal (DE)**

(74) Vertreter: **Grünbaum, Annekathrin et al Schnick & Garrels Patentanwälte PartG mbB Schonenfahrerstraße 7 18057 Rostock (DE)**

(56) Entgegenhaltungen:
**US-A1- 2004 057 334    US-A1- 2006 285 429**

**Beschreibung**

Die Erfindung bezieht sich auf ein Verfahren zur Messung des mittleren Gehalts einer Komponente in einem fließenden Stoffgemisch zweier oder mehrerer Komponenten, zum Beispiel bei der Befüllung eines Behälters, der durch zeitlich variablen Zufluss und zeitlich variablen Mengenanteil der Komponenten befüllt wird oder ist. Weiterhin bezieht sich die Erfindung auf die Bestimmung des mittleren Gehalts in dem Behälter. Dieser Messwert eignet sich als Istwert für die Regelung des mittleren Gehalts im Behälter während der Befüllung.

Das Verfahren eignet sich auch für die Messung des mittleren Gehalts einer Komponente des durch ein Rohr geflossenen Stoffgemisches über eine beliebige Zeit bei veränderlichen Zustandsbedingungen und veränderlichen momentanen Gehalten.

Bei einem Einsatz von Konzentrationsmessgeräten in Chemieanlagen wird der Momentanwert oder der Mittelwert des Gehaltes bzw. der Konzentration eines Säuregemisches bestimmt. In Durchflusssystemen lässt sich so bei gleichbleibendem Durchfluss ein mittlerer Gehalt (eine mittlere Konzentration) bestimmen. Sind während des Durchflusses von Stoffgemischen die Zusammensetzung und die Zustandsbedingungen nicht konstant, ist die exakte Bestimmung des mittleren Gehalts (der Konzentration) der gesamten Durchflussmenge nicht mehr zuverlässig.

Die Abfüllung und Verdünnung einer Säure aus einer Abfüllanlage in einen Transportbehälter mit einem vorgegebenen Säure-Soll-Gehalt wird beispielsweise realisiert, indem der Säuregehalt im Zulauf ständig gemessen wird und über regelungstechnische Prozesse versucht wird, den Soll-Gehalt durch Regelung des Zuflusses der Einzelkomponenten des Stoffgemisches zu erreichen. Insbesondere im Anlaufprozess kommt es hier zu Abweichungen zwischen Soll- und Ist-Gehalt. In einem solchen Regelungssystem ist allgemein davon auszugehen, dass während der Laufzeit des Misch- und Abfüllprozesses immer eine Regelabweichung existiert.

Im speziellen Fall sind aufwändige Regelmechanismen und -methoden erforderlich, die im Verlauf der Verdünnung einen konstanten Anteil der gewünschten Komponente sicher stellen, man ist aber nicht in der Lage die unausweichlichen Regelabweichungen so zu registrieren, dass der wahre mittlere Gehalt der Komponente der Mischung im Abfüllbehälter im Verlauf und Abschluss der Abfüllung korrekt ermittelbar wäre. Des Weiteren sind Verdünnungsvorgänge häufig von thermischen Effekten begleitet, deren Auswirkung auf die Temperaturänderungen, insbesondere bei Volumenstrommessungen, zu berücksichtigen sind und nach Messung kompensiert werden müssen.

Die Konzentration ist bei jeder Temperatur eine andere, da sich das Volumen ändert, was durch die Menge der Mischung eingenommen wird. Da beim Mischen von Stoffen meist Energie freigesetzt wird, ändert sich je nach Mischungsverhältnis auch die Temperatur und zusätzlich entspricht das Mischvolumen nicht der Summe der Ausgangsvolumina, je nach Stoffsystem wird eine Expansion oder Kontraktion registriert.

Bei Abfüllprozessen in Zusammenhang mit Verdünnungsprozessen werden häufig bekannte Medien über separate Zuleitungen an den Mischer geführt. Der Gehalt bzw. die Konzentration von zu verdünnenden Säuren in den Ausgangsbehältern ist oft bekannt. Die Gehaltsbestimmung und die Volumenstrom- bzw. Massenstrommessung erfolgt auch hier hinter dem Mischer.

Bekannt sind die Integration von Volumen- oder Massenstrom (es werden die im Abtastintervall angefallenen Teilvolumen und Teilmassen jeweils auf das Gesamtvolumen/Gesamtmasse addiert) in Durchfluss-Messsystemen, welche aber nur unter konstanter Zusammensetzung und konstanten Zustandsbedingungen oder unter konstanter Zusammensetzung zu zuverlässigen und genauen Ergebnissen führen können.

EP 1 725 839 B1 offenbart ein Coriolis-Massedurchflussmessgerät, bei dem die Messgeräte-Elektronik anhand des Erregerstroms und/oder anhand eines Anteils des Erregerstroms sowie unter Verwendung des Viskositäts-Messwerts zumindest zeitweise einen Konzentrations-Messwert ermittelt, der bei einem zwei- oder mehrphasigen Medium im Messrohr einen, insbesondere relativen, Volumen- und/oder Massenanteil einer Mediumsphase repräsentiert.

In DE 10 2014 115 566 A1 wird ein Messgerät mit einem integralen Messkanal zur Leitung eines Messmediums durch das Messgerät offenbart. An dem Messkanal sind wenigstens zwei Sensoren angeordnet, welche einerseits thermisch-physikalische Eigenschaften messen und andererseits mittels Vibration die Viskosität und/oder die Dichte des Messmediums bestimmen, welches durch den Messkanal vom ersten Sensor zum zweiten Sensor geführt wird. Aus diesen Daten werden die Konzentration, ein Masseanteil, ein Volumenanteil und/oder ein Partialdruck zumindest einer Komponente des mehrkomponentigen Messmediums ermittelt.

DE 10 2014 106 729 A1 beschreibt ein Verfahren zum Betreiben eines Messgerätes, insbesondere eines Durchfluss-Messgerätes, zur Bestimmung der Konzentration, des Volumenanteils, des Massenanteils und/oder des Partialdruckes zumindest einer Komponente in einem mehrkomponentigen Messmedium.

DE 10 2007 062 908 A1 offenbart ein Verfahren zur Bestimmung mindestens einer Prozessgröße eines strömenden Mediums, welches aus mehreren Komponenten besteht. Ein solches typisches Medium ist Biogas, welches im Wesentlichen aus den drei Komponenten Methan, Kohlendioxid und Wasserdampf besteht, und Spuren von Sauerstoff, Wasserstoff, Schwefelwasserstoff und Ammoniak enthält. Die Prozessgröße ist insbesondere eine Konzentration mindestens einer der Komponenten oder die Dichte des Mediums. Der Volumendurchfluss des Mediums wird mittels eines ersten Messprinzips, welches im Wesentlichen unabhängig von der Prozessgröße ist, gemessen. Und der Volumendurchfluss des Mediums wird mittels eines zweiten Messprinzips, welches von der Prozessgröße abhängig ist, gemessen. Aus mindestens den Messwerten des ersten und des zweiten Messprinzips wird die Prozessgröße bestimmt.

Verfahren zur Bestimmung des Gehalts einer Komponente in einem fließenden Stoffgemisch sind auch aus den Druckschriften US 2004/057334 A1 und US 2006/285429 A1 bekannt.

Um die Effektivität einer Biogas-Anlage nach DE 10 2007 062 908 A1 zu ermitteln und zu überwachen, ist es notwendig, den aktuellen Durchfluss und die aktuelle Konzentration der Komponenten des Biogases zu kennen. So bestimmt bspw. der Gehalt von Methan in dem Gasgemisch den Energiegehalt des Biogases. Allgemein sind Volumina je nach Stoffeigenschaft unterschiedlich stark von physikalischen Einflüssen wie Druck, Temperatur, Dichte etc. abhängig. Dies gilt vor allem für Gase und Gasgemische. Die Masse eines Stoffes hingegen ist unabhängig von diesen Einflüssen. Die Messung des Massedurchflusses erfolgt mit Coriolismessgeräten.

**Darstellung der Erfindung**

Es ist die Aufgabe der Erfindung, ein Verfahren zu entwickeln, die bei nicht konstanten Zustandsbedingungen, wie z.B. Temperatur und Druck wie auch den zeitlich veränderlichen Gehalt der einzelnen Zuflüsse, die Zuverlässigkeit und Genauigkeit der Abfüllung bezüglich der absoluten Massenbilanz und der Massenanteile der Komponenten in der Mischung nach erfolgter Verdünnung und Abfüllung sicherstellt. Im Besonderen soll eine vorgegebene Säure-Soll-Konzentration aus einer Abfüllanlage in einen Transportbehälter realisiert werden.

Sind während der Abfüllung und Herstellung von Stoffgemischen zweier Komponenten in einen Behälter die Zusammensetzung und die Zustandsbedingungen nicht konstant, muss zur exakten Bestimmung und Regulierung des Gehaltes der Komponenten der Mischung im Abfüllbehälter eine Methode verwendet werden, die sowohl die zeitlich veränderlichen Zustandsbedingungen wie auch den zeitlich veränderlichen Gehalt der einzelnen Zuflüsse berücksichtigt und die Integration von äquivalenten Einzelelementen zur Bilanzierung sicherstellt.

Die folgenden Begriffe werden im Sinne der Erfindung definiert:

-   Massenanteil gibt den relativen Anteil der Masse einer betrachteten Mischungskomponente an der Gesamtmasse des Stoffgemisches an;

-   Gehalt ist der Massenanteil eines bestimmten Stoffes in einem Stoffgemisch angegeben in Prozent;

-   mittlerer Gehalt ist der mittlere Massenanteil eines bestimmten Stoffes in einem Stoffgemisch über einen Befüllzeitraum, welcher gleichzeitig der Messzeitraum ist;

-   Stoffmenge ist als Basisgröße im Internationalen Einheitensystem (SI) ein allgemeiner Oberbegriff und gibt indirekt die Teilchenzahl einer Stoffportion an. Die Verwendung der Größe Stoffmenge ermöglicht die Betrachtung wägbarer Substanzmassen mit sehr hoher Teilchenzahl;

-   Zustandsbedingungen charakterisieren den Zustand eines Mediums für charakterisierende Größen wie Druck und Temperatur;

-   Ein Abtastintervall, hier bezeichnet mit $T$ ist der Zeitbereich zwischen zwei Messungen bzw. Abtastzeitpunkten, hier bezeichnet mit $t$ zur Bestimmung der Durchflussgrößen Volumenstrom und Massenstrom, der Temperatur und weiterer benötigter Zustandsmessgröße beginnend mit einem Abtastintervall $T_0$. Der zeitliche Abstand zwischen den zwei Abtastzeitpunkten $t_{i+1}$ und $t_i$ wird ausreichend klein gewählt, so dass über das sich ergebende Abtastintervall $T_i$ die Zustandsbedingungen und der Gehalt nahezu konstant sind;

-   Masseinkrement ist die Gesamtmasse des Stoffgemisches über ein Abtastintervall.

Die Lösung dieser Aufgabe besteht in der Verwendung der Messwerte des Gehalts und der Masse des Durchflusses über ein Abtastintervall zur Ermittlung der Komponentenmassen und deren separate Integration. Die Verwendung der Komponentenmassen gilt unter den üblichen Prozessbedingungen an jeder Stelle und unter allen Bedingungen, also vor allem unabhängig von Temperatur und Druck. Das Verhältnis der Gesamtmasse einer Komponente zur Gesamtmasse der Mischung spiegelt jederzeit den aktuellen Gehalt der gesamten gemessenen Durchflussmenge - hier speziell die Mischung im Behälter - wider. Der mittlere Gehalt des Gemisches im Behälter eignet sich besonders als Istwert-Eingangsgröße in einem Regelsystem zur Regelung der vorgegebenen Säure-Soll-Konzentration (Säure-Soll-Gehalt). In einem zweikomponentigen System genügt es, beide Komponentenmassen zu integrieren oder eine Komponentenmasse und die Gesamtmasse der Mischung.

Das erfindungsgemäße Verfahren zur Bestimmung eines mittleren Gehalts wenigstens einer Komponente in einem fließenden Stoffgemisch zweier oder mehrerer Komponenten in einem Behälter erfolgt zu jedem Abtastzeitpunkt in einem Befüllzeitraum. Der Behälter wird durch zeitlich variablen Zufluss aus wenigstens einer Zuleitung und mit momentanem, zeitlich variablem Gehalt der Komponenten befüllt. Die Bestimmung des mittleren Gehalts erfolgt durch:

a) Bestimmung einer geflossenen Masse (Masseninkrement) $m(T_i)$ des Stoffgemisches (SG) innerhalb ausreichend kleiner diskreter Abtastintervalle $T_i$ an Zeitpunkten $t_i$ beginnend beim Zeitpunkt $t_0$ durch

a1) Messung eines Volumenstroms $V$ und einer Dichte $\rho$ des Stoffgemisches (SG) und Bestimmung eines Massenstroms m des Stoffgemisches (SG) aus dem gemessenen Volumenstrom $V$ und der Dichte $\rho$ über ein Abtastintervall $T_i$ und Berechnung eines Masseinkrements $m(T_i)$
oder
a2) Messung des Massenstroms m des Stoffgemisches (SG) über ein Abtastintervall $T_i$ und Berechnung des Masseinkrements $m(T_i)$;

b) Bestimmung des momentanen Gehalts $G_K(T_i)$ als Massenanteil $w_K(T_i)$ des fließenden Stoffgemisches über ein Abtastintervall $T_i$ zugeordnet zur innerhalb dieses Abtastintervalls bestimmten Masseninkrements $m(T_i)$ des Stoffgemisches (SG)
dadurch gekennzeichnet, dass
c) eine Zerlegung des Masseinkrements $m(T_i)$ des Stoffgemisches (SG) über ein Abtastintervall $T_i$ in einzelne Komponentenmasseninkremente $m_K(T_i)$ aus dem Masseinkrement $m(T_i)$ des Stoffgemisches (SG) und dem ermittelten Massenanteil $w_K(T_i)$ einer Komponente (K) erfolgt;
d) eine separate Summierung aller Masseinkremente $m(T_i)$ des geflossenen Stoffgemisches (SG) und der ermittelten Komponentenmasseinkremente ($m_K(T_i)$) über den Befüllzeitraum ($t_0$ bis $t_n$) aus den Masseinkrementen $m(T_i)$ aller Abtastintervalle $T_i$ von $T_1$ bis $T_n$ zur Bestimmung der Masse $m_K$ der wenigstens einen Komponente (K) sowie der Bestimmung der Gesamtmasse $m_{ges}$ durchgeführt wird;
und
e) die Bestimmung des mittleren Gehalts $G_K$ der wenigstens einen Komponente (K) des geflossenen Stoffgemisches (SG) über den Befüllzeitraum $t_0$ bis $t_n$ durch Bestimmung des Massenanteils $w_K$ der wenigstens einen Komponente (K) im Behälter (5) über den Befüllzeitraum $t_0$ bis $t_n$ aus den Masseverhältnissen Masse $m_K$ der wenigstens einen Komponente (K) zur Gesamtmasse $m_{ges}$ erfolgt, wobei der mittlere Gehalt $G_K$ als der prozentuale Massenanteil $w_K$ der wenigstens einen Komponente (K) definiert ist.

In einer Ausführung wird der gemessene mittlere Gehalt einer Komponente als Istwert-Eingangsgröße für ein automatisches Mess- und Regelsystem zur Regelung des mittleren Gehalts der Komponenten zur Verfügung gestellt.

In einer weiteren Ausführung erfolgt die Messung des momentanen Gehalts und der Masse des Stoffgemisches über das Abtastintervall mittels eines Ultraschall- Durchfluss-Messsystems.

In einer weiteren Ausführung erfolgt die Berechnung und Integration der Komponentenmassen der Komponenten im Messumformer des Ultraschall- Durchfluss-Messsystems. Die Verrechnung und Integration des resultierenden mittleren Gehalts des gesamten durchgeflossen Stoffgemisches wird über einen geeigneten Prozessausgang bereitgestellt.

In einer weiteren Ausführung erfolgt bei mehreren Zuleitungen zu einem Mischer eine separate Summierung der Massen des geflossenen Stoffgemisches und der daraus ermittelten Komponentenmassen für jede Zuleitung zu jedem Abtastzeitpunkt und eine Zuordnung zu einem Messkanal. Die "totalisierten" Massen der einzelnen Messkanäle werden zum Abtastzeitpunkt erfasst, summiert und ins Verhältnis gesetzt.

In einer weiteren Ausführung erfolgt eine Einspeisung eines oder mehrerer Stoffgemische mit jeweils bekanntem konstantem Gehalt in je eine separate Zuleitung.

Vorteil der Erfindung ist es, dass aus dem Verhältnis der integrierten Komponentenmasse zur integrierten Gesamtmasse des Stoffgemisches über den Befüllzeitraum $t_0$ bis $t_n$ sich zu jedem Abtastzeitpunkt $t_i$ der mittlere Gehalt der Komponente korrekt bestimmen lässt.

Für ein Regelungssystem bietet sich die Verwendung des mittleren Gehaltes der Komponenten am Gesamtstoffgemisch als Eingangsgröße für den Gehalts-Istwert optimal an, weil er unverfälscht dem aktuellen Messergebnis entspricht und sich weiterhin der mittlere Gehalt im Behälter nur langsam ändert und damit nur geringe dynamische Anforderungen an das Regelungssystem stellt.

In einer speziellen Ausführung wird davon ausgegangen, dass der Gehalt der separat dem Mischungsprozess zugeführten Medien vor dem Mischer bekannt ist. Für diesen speziellen Fall genügt die separate Messung und die Integration der geflossenen Massen der Stoffgemische in den separaten Zuleitungen über den Befüllzeitraum.

Zu jedem Abtastzeitpunkt $t_i$ der Abfüllung erfolgt die Berechnung der jeweiligen aktuellen Komponentenmasse $m_K$ der Komponenten des Stoffgemisches in jeder separaten Zuleitung durch Multiplikation der Massen $m_{ges}$ der jeweiligen Zuleitung mit dem konstanten Gehalt der entsprechenden Komponente in der jeweiligen Zuleitung.

Zu jedem Abtastzeitpunkt $t_i$ der Abfüllung werden die Massen identischer Komponenten aus allen Zuleitungen zur jeweiligen Gesamtkomponentenmasse summiert und alle Gesamtkomponentenmassen zur Gesamtmasse des Stoffgemisches summiert, wobei diese Gesamtmasse der geflossenen Masse hinter dem Mischer entspricht.

Zu jedem Abtastzeitpunkt $t_i$ der Abfüllung lässt sich aus dem Verhältnis einer Gesamtkomponentenmasse zur Gesamtmasse aller Komponenten des Stoffgemisches aller Zuleitungen der mittlere Gehalt dieser Komponente der Abfüllung korrekt bestimmen.

In diesem speziellen Fall könnten mehr als zwei Komponenten zusammengeführt werden und der Gehalt aller Einzelkomponenten im Abfüllbehälter bestimmt werden. Eine Gehaltsmessung ist unter diesen speziellen Bedingungen nicht erforderlich.

## Ausführung der Erfindung

Die Erfindung wird anhand eines Ausführungsbeispiels erläutert. Hierzu zeigen

Figur 1    Schema einer Anordnung einer Ultraschall-Durchfluss- und Gehaltsmessung in einer Abfüllanlage hinter einem Mischer,

Figur 2    Schema einer Anordnung einer Ultraschall-Durchfluss- und Massenstrommessung in einer Abfüllanlage in Zuleitungen zum Mischer bei bekanntem konstanten Gehalt der Medien in den Zuleitungen.

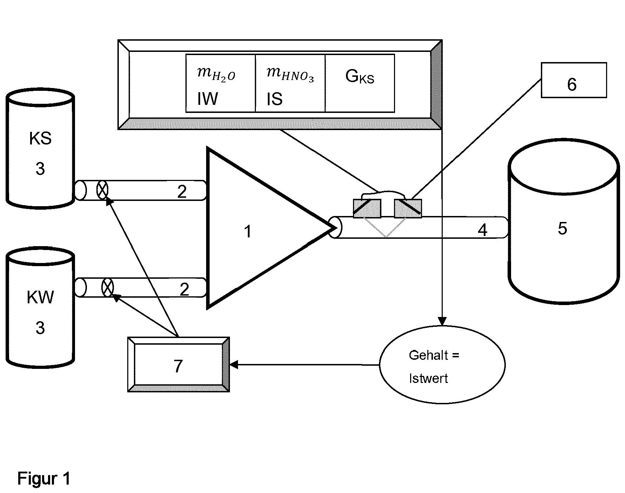

In Figur 1 wird ein Schema einer Anordnung einer Ultraschall-Durchflussund Gehaltsmessung in einer Abfüllanlage hinter einem Mischer (1) dargestellt. In der Abfüllanlage werden einem Mischer (1) jeweils über eine geregelte Zuleitung (2) zeitlich variabel verschiedene Komponenten K, beispielsweise eine Komponente Säure KS, z.B. Salpetersäure, und eine Komponente Wasser KW aus ihren Vorratsbehältern (3) beginnend beim Zeitpunkt $t_0$ zugeführt, wodurch hinter dem Mischer (1) für das Stoffgemisch SG ein zeitlich variabler Gehalt $G_K(T_i)$ resultiert. Hinter dem Mischer (1) sind am Zuleitungsrohr (4) zum Behälter (5) beispielsweise Clamp-On-Ultraschall-Sensoren (6) für eine Ultraschall-Durchflussmessung des Volumenstroms V und ein Temperatursensor zur Messung der Temperatur $\vartheta$ zu jedem Abtastzeitpunkt $t_i$ angebracht. Bei einer Vielzahl von binären Stoffsystemen stehen die Schallgeschwindigkeit c und die Temperatur $\vartheta$ in einem definierten Verhältnis zum Gehalt $G_K(T_i)$ und der Dichte $\rho$ und sind deshalb vorteilhaft für die Bestimmung des Massenstroms m des Gemischs (aus dem Volumenstrom) sowie für die Messung des Gehalts $G_K(T_i)$ des Stoffgemisches SG aus Wasser KW und Säure KS zu jedem Abtastzeitpunkt $t_i$ verwendbar. Aus dem gemessenen Massenstrom ergibt sich das Masseninkrement $m(T_i)$ des Durchflusses über das Abtastintervall $T_i$. Anhand des Gehalts $G_K(T_i)$ wird das Masseninkrement $m(T_t)$ des Stoffgemisches SG in die Komponentenmassen Wasser $m_{H_2O}(T_i)$ und Säure $m_{HNO_3}(T_i)$ zerlegt. Die Komponentenmassen Wasser $m_{H_2O}(T_i)$ und Säure $m_{HNO_3}(T_i)$ über ein Abtastintervall $T_i$ werden jeweils einem Integrator für Wasser IW und für Säure IS über den Befüllzeitraum $t_0$ bis $t_n$ zugeführt.

Zu jedem Abtastzeitpunkt $t_i$ der Abfüllung ergibt sich der mittlere Gehalt $G_{KS}$ der Säure KS im Behälter (5) aus dem Verhältnis der Komponentenmasse Säure $m_{HNO_3}$ zur Summe der Komponentenmassen Wasser $m_{H_2O}$ und Säure $m_{HNO_3}$. Über einen geeigneten Prozessausgang, z.B. eine Stromschleife, am Integrator IW, IS kann der aktuelle Ist-Wert des mittleren Gehalts $G_{KS}$ der Säure KS im Behälter (5) einem Regelungssystem (7) zugeführt werden, welches die Zuflüsse in den Zuleitungen (2) der Komponenten Säure KS und Wasser KW zum Mischer (1) regelt.

Für das erfindungsgemäße Verfahren zur Bestimmung des mittleren Gehalts $G_{KS}$ mindestens einer Komponente K in dem Stoffgemisch SG in dem Behälter (5), der durch zeitlich variablen Zufluss und zeitlich variablem Mengenanteil der Komponenten K befüllt wird oder ist, kann einerseits ein Messsystem zur Messung des Momentanwertes des Gehalts $G_K(T_i)$ des (zweikomponentigen) Stoffgemischs SG in Zusammenhang mit einer Messung des Massenstroms m zur Anwendung kommen. Alternativ kann sich die Messung des Massenstroms m auch aus einer Verrechnung eines gemessenen Volumenstromes V unter Einbeziehung von Dichte $\rho$ ergeben. Es wird im Ergebnis einer Durchflussund Gehaltsmessung ein Gehalt $G_K(T_i)$ über ein Gesamtmasseninkrement $m(T_i)$ des Durchflusses in dem Abtastintervall $T_i$ ermittelt. Insbesondere ist die Zusammenführung der Masseintegration der Komponenten K mit der Gehaltsmessung von Bedeutung. Über ein Abtastintervall $T_i$ wird in Folge immer der Gehalt $G_K(T_i)$ in Zusammenhang mit der dazugehörigen Masse $m(T_i)$ des Stoffgemisches SG betrachtet.

Die Messung eines Volumenstroms V und der Masse m des durchgeflossenen Stoffgemisches SG sowie die Bestimmung des Gehalts $G_K(T_i)$ erfolgt durch diskrete Abtastung der Einzelmessgrößen "Volumenstrom" und "Gehalt" über den Befüllzeitraum $t_0$ bis $t_n$, beginnend mit $t_0$, in ausreichend kleinen Zeitabständen zur Ermittlung der Mittelwerte der Messgrößen über das jeweilige Abtastintervall $T_i$. Dadurch wird eine ausreichend hohe zeitliche Auflösung erreicht. Es wird angenommen, dass über ein ausreichend kleines Abtastintervall $T_i$ die Zustandsbedingungen und der Gehalt $G_K(T_i)$ nahezu konstant sind.

Anhand des ermittelten Gehalts $G_K(T_i)$ des Stoffgemisches SG wird die über das Abtastintervall $T_i$ gemessene

Masse $m(T_i)$ des Stoffgemisches SG in die Komponentenmasse Wasser $m_{H2O}$ und die Komponentenmasse Säure $m_{HNO3}$ zerlegt. Der Gehalt $G_K(T_i)$ spiegelt den Anteil der Komponentenmasse $m_K(T_i)$ einer Komponente K an der Gesamtmasse $m_i$ in Prozent wieder. Hier gilt:

$$G_K(T_i) = \frac{m_K(T_i)}{m_i} \cdot 100$$

(alle Größen über das Abtastintervall $T_i$)

Anschließend werden die ermittelten Komponentenmassen Wasser $m_{H2O}$ und Säure $m_{HNO3}$ und/oder die Masse m des Stoffgemisches SG über das Abtastintervall $T_i$ zu der Gesamtmasse $m_{KH2O}$ und $m_{KHNO3}$ der Komponenten K und/oder der Gesamtmasse $m_{ges}$ des Stoffgemisches SG summiert. Dies erfolgt unabhängig von Temperatur und Druck.

Das Verhältnis der Gesamtmasse $m_K$ einer Komponente K zur Gesamtmasse $m_{ges}$ des Stoffgemisches SG spiegelt zu jedem Abtastzeitpunkt $t_i$ den aktuellen Gehalt $G_K$ der gesamten gemessenen Durchflussmenge - hier speziell das Stoffgemisch SG im Behälter (5) - wieder. Wegen der Verwendung der Massen m ist das Verfahren zuverlässig bei Zustandsänderungen, Gehaltsänderungen oder Änderungen der Fließgeschwindigkeit über den gesamten Abfüllvorgang.

Der über das Messverfahren ermittelte mittlere Gehalt $G_K$ im Behälter (5) steht über einen gesamten Auffüllvorgang entsprechend der Genauigkeit des Messverfahrens zur Verfügung und kann dem Regelungssystem (7) als Eingangswert zur Verfügung gestellt werden. In Abhängigkeit von der Dynamik und der Länge des Auffüllvorganges ändert sich der mittlere Gehalt $G_K$ der gesamten Durchflussmenge des Stoffgemisches SG im Behälter (5) im Vergleich zu den Messwerten über ein Abtastintervall nur langsam.

Die Berechnung in einem Ultraschalldurchflussmessgerät mit Gehaltsmessung erfolgt mit den folgenden Formeln:

Der Massenanteil w (verwendet als Äquivalent zum Gehalt G) wird durch Messung der Temperatur $\vartheta$ und der Schallgeschwindigkeit c des fließenden Stoffgemisches SG bestimmt.

$$w_K = f(\vartheta, c) \qquad\qquad\qquad \text{Gl. (1)}$$

Die Dichte wird durch Messung von Temperatur $\vartheta$ und aus dem Massenanteil $w_K$ nach Gleichung 1 bestimmt.

$$\rho = f(\vartheta, w_K) \qquad\qquad\qquad \text{Gl. (2)}$$

Die Masse m des geflossenen Stoffgemisches SG über ein beliebiges Abtastintervall $T_i$ kann anhand des Massenstroms m bestimmt werden. Sie kann aber auch unter Verwendung von Volumenstrom V bzw. Volumen V und Dichte $\rho$ direkt berechnet werden, oder nach einer beliebigen anderen nach Stand der Technik verfügbaren Methode ermittelt werden. Verwendbare Methoden zur Ermittlung der geflossenen Stoffgemischmasse m über ein beliebiges Abtastintervall $T_i$ und die Bestimmung des zugehörigen Gehalts G bzw. des Massenanteils $w_K$ der Komponenten K dieses Stoffgemisches SG werden als bekannt vorausgesetzt.

Die gemessene Masse m des geflossenen Stoffgemisches SG über ein Abtastintervall $T_i$ wie auch die dazu ermittelte Komponentenmasse $m_K$ über das Abtastintervall $T_i$ nennen wir hier Masseinkrement $m(T_i)$ des Stoffgemisches SG bzw. Masseinkrement $m_K(T_i)$ der Komponente K.

Varianten zur Bestimmung der geflossenen Masse über ein Abtastintervall:

1. Aus dem mittleren Volumenstrom V des Stoffgemisches SG über ein Abtastintervall $T_i$ und der Dichte $\rho$ wird die Masse m des Stoffgemisches SG über das Abtastintervall $T_i$ nach Gleichung 3.1 bestimmt.

$$m(T_i) = V(T_i) \cdot \rho(T_i) \cdot T_i \qquad\qquad\qquad \text{Gl. (3.1)}$$

mit

$$V(T_i) = \frac{\left(V(t_i) + V(t_{i-1})\right)}{2}$$

und

$$V(T_i) = V(T_i) \quad T_i$$

und

$$\rho(T_i) = \frac{(\rho(t_i)+\rho(t_{i-1}))}{2}$$

2. Der Massenstrom m des Stoffgemisches SG kann aus dem gemessenen Volumenstrom V und der Dichte $\rho$ nach Gleichung 3.2 bestimmt werden:

$$m = V \quad \rho \qquad\qquad Gl. (3.2.)$$

Über ein Abtastintervall $T_i$ gilt dann:

$$m\,(T_i) = V(T_i) \quad \rho(T_i) \qquad\qquad Gl. (3.3.)$$

$$\text{mit } T_i = t_i \quad t_{i-1}$$

,wobei $T_i$ beginnend bei $T_1$

3. Der Massenstrom m des Stoffgemisches SG über ein Abtastintervall $T_i$ kann alternativ direkt mit einem geeigneten Massenstrommessgerät gemessen werden.

$$m(T_i) = \frac{(m(t_i)+m(t_{i-1}))}{2} \qquad\qquad Gl. (3.4.)$$

Das Masseinkrement $m(T_i)$ der Gesamtmasse des Stoffgemischs SG über das Abtastintervall $T_i$ wird bei der Massenstrommessung über Gleichung 3.5 bestimmt:

$$m(\,T_i\,) = m(T_i) \quad T_i \qquad\qquad Gl. (3.5.)$$

Die Zerlegung des Masseinkrements $m(T_i)$ des Stoffgemisches SG über ein Abtastintervall $T_i$ in einzelne Komponentenmasseninkremente $m_K(T_i)$ ist ein wichtiger Schritt in dem erfindungsgemäßen Verfahren. Das Masseinkrement $m_K(T_i)$ einer Komponente K im Abtastintervall $T_i$ ergibt sich nach Gleichung 4 aus der Stoffgemischmasse $m_{ges}$ und dem ermittelten Massenanteil $w_K$:

$$m_K(T_i) = m(T_i) \quad w_K(T_i) \qquad\qquad Gl. (4)$$

Die Summe aller Massenanteile eines Gemisches ist 1:

$$1 = \sum_{k=1}^{n} w_K \qquad\qquad Gl. (4.1)$$

Bei einer Massenstrommessung wäre eine Modifikation der Zerlegung der Masseinkremente möglich, indem zuerst der Massenstrom $m_K$ der Komponente K über ein Abtastintervall $T_i$ anhand des ermittelten Massenanteils $m_K$ berechnet wird und anschließend die Komponentenmasse $m_K$ und die Stoffgemischgesamtmasse $m_{ges}$ aus den Massenströmen m bestimmt werden:

Für Massenstrom m des Stoffgemisches SG über ein Abtastintervall $T_i$ gelten die Gleichungen 3.3 und 3.4.

Der Komponentenmassenstrom $m_K$ über ein Abtastintervall $T_i$ ergibt sich analog Gleichung 4 nach Gleichung 4.2:

$$m_K(T_i) = m(T_i) \quad w_K(T_i) \qquad \text{Gl. (4.2)}$$

Das Masseinkrement der Komponentenmasse über ein Abtastintervall wird analog zu Gleichung 3.5 aus dem Komponentenmassenstrom berechnet:

$$m_K(T_i) = m_K(T_i) \quad T_i \qquad \text{Gl. (4.3)}$$

Diese Formeln gelten für jeden Zeitpunkt $t$ bzw. Zeitraum $T$, für jede Komponente K und für jede Zuleitung (2), die zur Bilanz beiträgt. Es wird die Bilanzsumme für eine Zuleitung 2, eine Komponente K und einen Befüllzeitraum $t_0$ bis $t_n$ gebildet.

Ausführungsbeispiel für einen Messkanal, zwei Komponenten und Summierung der Einzelmassen:

Die Messung und Berechnung des Massenanteils $w$, der Dichte $\rho$ und des Massenstroms $m$ über das Abtastintervall $T_i$ erfolgt am Ende des Abtastintervalls entsprechend Gleichungen (1), (2) und (3.1) - (4.2) zum Zeitpunkt $t_i$.

In jedem Abtastintervall $T_i$ des Messkanals berechnet sich das Masseinkrement der ersten Komponente in einem zweikomponentigen Stoffgemisch analog Gleichung (4) für Komponente K1 nach Gleichung (5.1):

$$m_{K1}(T_i) = m(T_i) \quad w_{K1}(T_i) \qquad \text{Gl. (5.1)}$$

analog dazu das Masseinkrement der zweiten Komponente nach Gleichung (5.2):

$$m_{K2}(T_i) = m(T_i) \quad (1 \quad w_{K1}(T_i)) \qquad \text{Gl. (5.2)}$$

Die separate Summierung der Masseinkremente $m(T_i)$ des geflossenen Stoffgemisches SG und der daraus ermittelten Komponentenmasseinkremente $m_K(T_i)$ ist ein weiterer wichtiger Schritt in dem Verfahren.

Bestimmung der Masse m Komponente K1 über den Befüllzeitraum $t_0$ bis $t_n$ über die Masseinkremente $m(T_i)$ aller Abtastintervalle bis $T_n$ (Abtastzeitpunkt $t_0$)

$$m_{K1} = \sum_{i=1}^{n} m_{K1}(T_i) \qquad \text{Gl. (6.1)}$$

analog gilt für die zweite Komponente des zweikomponentigen Stoffgemisches SG

$$m_{K2} = \sum_{i=1}^{n} m_{K2}(T_i) \qquad \text{Gl. (6.2)}$$

und analog gilt für die Gesamtmasse $m_{ges}$ im Messkanal und für Gesamtmasse im Befüllzeitraum $t_0$ bis $t_n$ über alle Abtastintervalle

$$m_{ges} = \sum_{i=1}^{n} m(T_i) \qquad \text{Gl. (6.3)}$$

$$= m_{K1} + m_{K2} \qquad \text{Gl. (6.4)}$$

Der zu bestimmende Massenanteil bzw. der Gehalt G einer Komponente K des geflossenen Stoffgemisches SG über einen Befüllzeitraum $t_0$ bis $t_n$ ist ein weiterer Bestandteil des Verfahrens:

Die Bestimmung des Massenanteils der ersten Komponente K1 im Behälter 5 über den Befüllzeitraum $t_0$ bis $t_n$ ergibt sich nach Gleichung 3 aus den Masseverhältnissen

$$w_{K1} = \frac{m_{K1}}{m_{ges}} \qquad \text{Gl. (7)}$$

Der abgeleitete Gehalt G der ersten Komponente K1 über den Befüllzeitraum $t_0$ bis $t_n$ ist der Massenanteil in %

$$G_{K1} = w_{K1} \quad 100\%$$ Gl. (8)

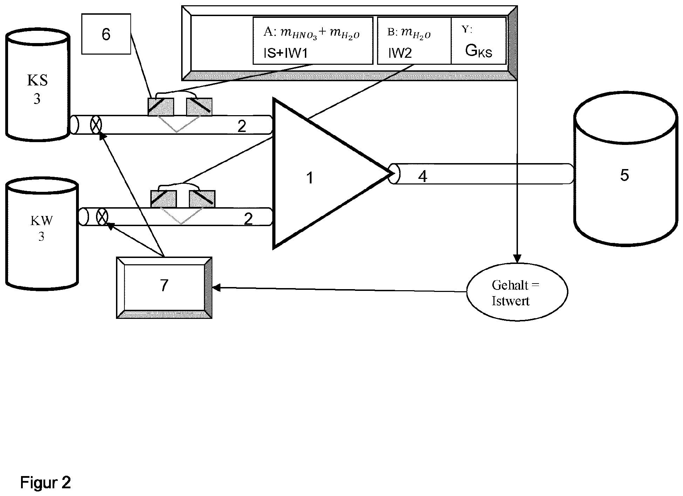

Ein weiteres Ausführungsbeispiel wird in Figur 2 in einem Schema zur Anordnung einer Ultraschall-Durchfluss- und Massenstrommessung in der Abfüllanlage in den Zuleitungen (2) zum Mischer (1) gezeigt.

In der Abfüllanlage werden dem Mischer (1) eine Komponente K in Form einer Säure KS mit dem zeitlich variablen Gehalt $G_K(T_i)$ und eine Komponente K in Form von Wasser KW aus ihren Vorratsbehältern (3) zugeführt. An den separaten Zuleitungen (2) zum Mischer (1) sind beispielsweise Clamp-On-Ultraschall-Sensoren (6) für eine Ultraschall-Durchflussmessung des Massenstroms m des Gemischs sowie für die Messung des Gehalts $G_K(T_i)$ des Stoffgemisches SG aus Wasser KW und Säure KS zu jedem Abtastzeitpunkt $t_i$ in jedem Rohr angeordnet. Aus dem gemessenen Massenstrom ergeben sich die Massen $m_i$ des Durchflusses über das Abtastintervall $T_i$ in jeder separaten Zuleitung. Anhand des Gehalts $G_K(T_i)$ in jeder separaten Zuleitung werden die Massen $m_i$ des Stoffgemisches SG in jeder Zuleitung in die Komponentenmassen Wasser $m_{H2O}$ und Säure $m_{HNO3}$ zerlegt. Die Annahme einer Komponente Wasser KW in der zweiten Zuleitung stellt hier eine Vereinfachung dar. Das Verfahren gilt hier auch, wenn in dieser zweiten Zuleitung ein zweikomponentiges Stoffgemisch vorhanden wäre.

Die Komponentenmassen Wasser $m_{H2O}$ und Säure $m_{HNO3}$ über ein Abtastintervall $T_i$ werden jeweils einem Integrator für Wasser IW und für Säure IS in jeder separaten Zuleitung über den Befüllzeitraum $t_0$ bis $t_n$ zugeführt.

Damit ergeben sich zu jedem Abtastzeitpunkt $t_i$ eine StoffgemischGesamtmasse $m_{ges}$ durch Addition aller integrierten Einzelmassen aller Komponenten aller Zuleitungen und mindestens eine Komponentenmasse $m_K$ , hier im Beispiel die Komponente Säure mit der Masse $m_{HNO3}$ , durch Addition aller bis zum Abtastzeitpunkt $t_i$ in allen separaten Zuleitungen integrierten Komponentenmassen $m_{HNO3}$ (Knr).

Zu jedem Abtastzeitpunkt $t_i$ der Abfüllung ergibt sich der mittlere Gehalt $G_{KS}$ der Säure KS im Behälter (5) aus dem Verhältnis der Komponentenmasse Säure $m_{HNO3}$ aller Zuleitungen zur Gesamtmasse $m_{ges}$ des Stoffgemischs aller Zuleitungen. Über einen geeigneten Prozessausgang, z.B. eine Stromschleife, kann der aktuelle Ist-Wert des mittleren Gehalts $G_{KS}$ der Säure KS im Behälter (5) einem Regelungssystem (7) zugeführt werden, welches die Zuflüsse in den Zuleitungen zum Mischer (1) regelt

Als Ausführungsbeispiel wird hier eine Messung in mehreren Messkanälen vorgestellt. Die Messung erfolgt in den Zuleitungen (2) zu einem Mischer (1). Es wird hier eine separate Summierung der Massen m des geflossenen Stoffgemisches SG und der daraus ermittelten Komponentenmassen $m_K$ aus allen beteiligten Messkanälen betreffend alle dem Mischer zugeführten Zuleitungen (2) zu jedem Abtastzeitpunkt t vorgenommen. Die Einzelmessungen in den Messkanälen erfolgen entsprechend den Formeln (1) bis (6.4)

Die Summenelemente werden dem Messkanal zugeordnet:

Beispiel Messkanal A Summe aller Komponentenmassen $m_{K1}$ der Komponente K1 zum Abtastzeitpunkt $t_n$ in Kanal A

$$m_{K1}(A) = \sum_{i=1}^{n} m_{K1}(T_i) \ (A)$$ Gl. (9.1)

Summe aller Komponentenmassen $m_{K2}$ der Komponente K2 zum Abtastzeitpunkt $t_n$ in Kanal A

$$m_{K2}(A) = \sum_{i=1}^{n} m_{K2}(T_i) \ (A)$$ Gl. (9.2)

Summe aller Stoffgemischmassen $m_{ges}$ zum Abtastzeitpunkt $t_n$ gemessen in Kanal A für ein zweikomponentiges Stoffgemisch SG:

$$m_{ges}(A) = \sum_{i=1}^{n} m(T_i) \ (A)$$ Gl. (9.3)
$$= m_{K1}(A) + m_{K2}(A)$$

Bei der Anwendung auf mehrere Messkanäle gelten Abtastintervalle jeweils für den betrachteten Kanal. Aus übergeordneter Sicht der Kanalverrechnung werden die "totalisierten" Massen der Kanäle zum Abtastzeitpunkt der Verrechnung erfasst, summiert und ins Verhältnis gesetzt. Für ein zweikomponentiges Stoffgemisch gilt über alle Messkanäle A - j, wobei jeder Messkanal eine eigene Zuleitung im Sinne der Massenbilanz repräsentiert: Summe aller Komponentenmassen $m_{K1}$ der Komponente K1 über alle Messkanäle zum Abtastzeitpunkt $t_n$

$$m_{K1}(A \quad j) = \sum_{Knr=A}^{j} m_{K1}(Knr)$$ 
Gl. (10.1)

Summe aller Komponentenmassen $m_{K2}$ der Komponente K2 über alle Messkanäle zum Abtastzeitpunkt $t_n$

$$m_{K2}(A \quad j) = \sum_{Knr=A}^{j} m_{K2}(Knr)$$ 
Gl. (10.2)

Summe aller Stoffgemischmassen über alle Messkanäle zum Abtastzeitpunkt $t_n$

$$m_{ges}(A \quad j) = \sum_{Knr=A}^{j} m_{ges}(Knr)$$
$$= m_{K1}(A \quad j) + m_{K2}(A \quad j)$$
Gl. (10.3)

Massenanteil der Komponente K1 über alle Messkanäle über den Befüllzeitraum $t_0$ bis $t_n$:

$$w_{K1}(A \quad j) = \frac{m_{K1}(A-j)}{m_{ges}(A-j)}$$ 
Gl. (11)

Gehalt $G_{K1}$ der Komponente K1 über alle Messkanäle über den Befüllzeitraum $t_0$ bis $t_n$:

$$G_{K1}(A \quad j) = w_{K1}(A \quad j) \quad 100\%$$ 
Gl. (12)

Die Berechnung für das konkrete Ausführungsbeispiel erfolgt mit den folgenden Formeln:

Massenanteil über ein Abtastintervall

$$w_{HNO_3} = f(\vartheta, c)$$

$$\rho = f(\vartheta, w_{HNO_3})$$

Massenstrom Stoffgemisch über ein Abtastintervall

$$m = V \quad \rho$$

Massenstrom Säure über ein Abtastintervall

$$m_{HNO_3} = V \quad \rho \quad w_{HNO_3}$$

Massenstrom Wasser über ein Abtastintervall

$$m_{H_2O} = V \quad \rho \quad (1 \quad w_{HNO_3})$$

Summe aller Komponentenmassen der Säure zum Zeitpunkt $t_n$

$$m_{HNO_3} = \sum_{t=1}^{t=n} m_{HNO_3}(t) \quad t$$

Massenanteil Säure über den Befüllzeitraum $t_n$

$$w_{HNO_3} = \frac{m_{HNO_3}}{m_{HNO_3} + m_{H_2O}}$$

$$G = w_{HNO_3} \quad 100\%$$

| | | |
|---|---|---|
| G | Gehalt in % | |
| w | Massenbruch (Massenanteil, Massengehalt, Massenmenge von Komponenten in einem Stoffgemisch) | |
| $T$ | Temperatur | |
| $c$ | Schallgeschwindigkeit | |
| $\rho$ | Dichte | |
| $m$ | Masse | |
| $m$ | Massenstrom | |
| $V$ | Volumen | |
| $V$ | Volumenstrom | |
| Index | bezeichnet bei Gehaltsangaben die Komponente (Analyt) bezeichnet bei sonstigen Größen den Anteil, der durch genau den einen Stoff erbracht wird | |

**Bezugszeichen**

| | |
|---|---|
| 1 | Mischer |
| 2 | Zuleitung |
| 3 | Vorratsbehälter |
| 4 | Zuleitungsrohr |
| 5 | Behälter |
| 6 | Clamp-On-Ultraschall-Sensoren |
| 7 | Regelungssystem |

| | |
|---|---|
| IW | Integrator Wasser |
| IS | Integrator Säure |
| K | Komponenten |
| KS | Säure |
| KW | Wasser |
| SG | Stoffgemisch |

| | |
|---|---|
| $\rho$ | Dichte |
| $\vartheta$ | Temperatur |
| A, j | Messkanal A bis Messkanal j, wobei j Index für Kanalnummer ist |
| $c$ | Schallgeschwindigkeit |
| $G$ | Gehalt in % |
| $G_K$ | Gehalt einer Komponente K über den Abfüllzeitraum |
| $G_{KS}$ | Gehalt der Säure KS |
| $G_K(T_i)$ | Gehalt einer Komponente K über ein Abtastintervall |
| Knr | Mess-Kanalnummer, bezeichnet einen Messkanal bei paralleler Messung in mehreren Zuleitungen zu einem Mischer |
| $m$ | Massenstrom |
| $m_{H_2O}$ | Komponentenmasse Wasser |
| $m_{H_2O}(T_i)$ | Masseninkrement Wasser |
| $m_{HNO_3}$ | Komponentenmasse (Salpeter-)Säure |
| $m_K$ | Komponentenmasse einer Komponente K |
| $m_K\,(Knr)$ | Komponentenmasse einer Komponente K bezogen auf einen Messkanal |
| $m_K(T_i)$ | Masseninkrement, Komponentenmasse einer Komponente K über ein Abtastintervall |
| $m_{ges}$ | Gesamtmasse des Stoffgemisches über den Befüllzeitraum $t_0$ bis $t_n$ |
| $m_{ges}\,(Knr)$ | Gesamtmasse des Stoffgemisches über den Befüllzeitraum $t_0$ bis $t_n$ bezogen auf einen Messkanal |
| $m(T_i)$ | Masseninkrement, Gesamtmasse des Stoffgemisches über ein Abtastintervall |

$t_0, t_i, t_n$      Abtastzeitpunkt, zugehörig zu einem Abtastintervall, beginnend mit $t_0$, $t_i$ ist der betrachtete Abtastzeitpunkt, $t_n$ bezeichnet den letzten Abtastzeitpunkt innerhalb des Befüllzeitraums $t_0$ bis $t_n$

$T_1, T_i, T_n$      Abtastintervall, beginnend mit $T_1$, $T_i$ ist das betrachtete Abtastintervall, $T_n$ bezeichnet das letzte Abtastintervall innerhalb des Befüllzeitraums $t_0$ bis $t_n$

$V$      Volumen

$V(T_i)$      Volumeninkrement, Volumen des Stoffgemisches über ein Abtastintervall

$V$      Volumenstrom

$w$      Massenanteil von Komponenten in einem Stoffgemisch

$w_K(T_i)$      Masseanteil einer Komponente über ein Abtastintervall

## Patentansprüche

1. Verfahren zur Bestimmung eines mittleren Gehalts ($G_K$) wenigstens einer Komponente (K) in einem fließenden Stoffgemisch (SG) zweier oder mehrerer Komponenten in einem Behälter (5) zu jedem Abtastzeitpunkt ($t_i$) in einem Befüllzeitraum ($t_0$ bis $t_n$),

   wobei der Behälter (5) durch zeitlich variablen Zufluss aus wenigstens einer Zuleitung (2) und mit momentanem, zeitlich variablem Gehalt ($G_K(T_i)$) der Komponenten (K) befüllt wird,
   durch

   a) Bestimmung einer geflossenen Masse ($m_i$) des Stoffgemisches (SG) innerhalb ausreichend kleiner diskreter Abtastintervalle ($T_i$) an Zeitpunkten ($t_i$) beginnend beim Zeitpunkt ($t_0$) durch

   a1) Messung eines Volumenstroms (V) und einer Dichte (p) des Stoffgemisches (SG) und Bestimmung eines Massenstroms (m) des Stoffgemisches (SG) aus dem gemessenen Volumenstrom (V) und der Dichte ($\rho$) über ein Abtastintervall ($T_i$) und Berechnung eines Masseinkrements ($m(T_i)$);
   oder
   a2) Messung des Massenstroms (m) des Stoffgemisches (SG) über ein Abtastintervall ($T_i$) und Berechnung des Masseinkrements ($m(T_i)$);

   b) Bestimmung des momentanen Gehalts ($G_K(T_i)$) als Massenanteil ($w_K$) des fließenden Stoffgemisches über ein Abtastintervall ($T_i$) zugeordnet zur innerhalb dieses Abtastintervalls bestimmten Masse ($m_i$) des Stoffgemisches (SG) **dadurch gekennzeichnet, dass**
   c) eine Zerlegung des Masseinkrements ($m(T_i)$) des Stoffgemisches (SG) über ein Abtastintervall ($T_i$) in einzelne Komponentenmasseninkremente ($m_K(T_i)$) aus der Masse ($m_i$) des Stoffgemisches (SG) und dem ermittelten Massenanteil ($w_K$) einer Komponente (K) erfolgt;
   d) eine separate Summierung aller Masseinkremente ($m(T_i)$) des geflossenen Stoffgemisches (SG) und der ermittelten Komponentenmasseinkremente ($m_K(T_i)$) über den Befüllzeitraum ($t_0$ bis $t_n$) aus den Masseinkrementen ($m(T_i)$) aller Abtastintervalle ($T_i$ von $T_1$ bis $T_n$) zur Bestimmung der Masse ($m_K$) der wenigstens einen Komponente (K) sowie der Bestimmung der Gesamtmasse ($m_{ges}$) durchgeführt wird;
   und
   e) die Bestimmung des mittleren Gehalts ($G_K$) der wenigstens einen Komponente (K) des geflossenen Stoffgemisches (SG) über den Befüllzeitraum ($t_0$ bis $t_n$) durch Bestimmung des Massenanteils ($w_K$) der wenigstens einen Komponente (K) im Behälter (5) über den Befüllzeitraum ($t_0$ bis $t_n$) aus den Masseverhältnissen Masse ($m_K$) der wenigstens einen Komponente (K) zur Gesamtmasse ($m_{ges}$) erfolgt, wobei der mittlere Gehalt ($G_K$) als der prozentuale Massenanteil ($w_K$) der wenigstens einen Komponente (K) definiert ist.

2. Verfahren nach Anspruch 1 **dadurch gekennzeichnet, dass** der gemessene mittlere Gehalt ($G_K$) einer Komponente als Istwert-Eingangsgröße für ein automatisches Mess- und Regelsystem zur Regelung des mittleren Gehalts ($G_K$) der Komponenten (K) zur Verfügung gestellt wird.

3. Verfahren nach Anspruch 1 **dadurch gekennzeichnet, dass** die Messung des momentanen Gehalts ($G_K(T_i)$) und der Masse ($m_i$) des Stoffgemisches (SG) über das Abtastintervall ($T_i$) mittels eines Ultraschall-Durchfluss-Messsystems erfolgt.

4. Verfahren nach Anspruch 3 **dadurch gekennzeichnet, dass** die Berechnung und Integration der Komponenten-

massen ($m_K(T_i)$)der Komponenten (K) im Messumformer des Ultraschall-Durchfluss-Messsystems erfolgt und die Verrechnung und Integration des resultierenden mittleren Gehalts ($G_K$) des gesamten durchgeflossen Stoffgemisches (SG) über einen geeigneten Prozessausgang bereitgestellt wird.

5. Verfahren nach einem der Ansprüche 1 bis 4 **dadurch gekennzeichnet, dass** bei mehreren Zuleitungen (2) zu einem Mischer (1) eine separate Summierung der Massen (m) des geflossenen Stoffgemisches (SG) und der daraus ermittelten Komponentenmassen ($m_k$) für jede Zuleitung (2) zu jedem Abtastzeitpunkt ($t_n$) erfolgt und einem Messkanal (Knr) zugeordnet werden, wobei "totalisierte" Massen (m) der einzelnen Messkanäle (Knr) zum Abtastzeitpunkt ($t_n$) erfasst, summiert und ins Verhältnis gesetzt werden.

6. Verfahren nach Anspruch 5 **dadurch gekennzeichnet, dass** eine Einspeisung eines oder mehrerer Stoffgemische (SG) mit jeweils bekanntem konstantem Gehalt (G) in je eine separate Zuleitung erfolgt.

**Claims**

1. Method for determining a mean content ($G_K$) of at least one component (K) in a flowing substance mixture (SG) of two or more components in a container (5) at any sampling time ($t_i$) during a filling time period ($t_0$ to $t_n$),

   wherein the container (5) is filled by means of a time-varying inflow from at least one feed line (2) and with instantaneous, time-varying content ($G_K(T_i)$) of the components (K),
   by

   a) determining a mass ($m_i$) of the substance mixture (SG) that has flowed, said determination being implemented within sufficiently small discrete sampling intervals ($T_i$) at times ($t_i$) starting at the time ($t_0$) by

   a1) measuring a volumetric flow rate (V) and a density (p) of the substance mixture (SG) and determining a mass flow rate (m) of the substance mixture (SG) from the measured volumetric flow rate (V) and the density ($\rho$) over a sampling interval ($T_i$) and calculating a mass increment ($m(T_i)$);
   or
   a2) measuring the mass flow rate (m) of the substance mixture (SG) over a sampling interval ($T_i$) and calculating the mass increment ($m(T_i)$);

   b) determining the current content ($G_K(T_i)$) as a mass fraction ($w_K$) of the flowing substance mixture over a sampling interval ($T_i$) assigned to the mass ($m_i$) of the substance mixture (SG) determined within this sampling interval, **characterized in that**
   c) there is a decomposition of the mass increment ($m(T_i)$) of the substance mixture (SG) over a sampling interval ($T_i$) into individual component mass increments ($m_K(T_i)$) from the mass ($m_i$) of the substance mixture (SG) and the determined mass fraction ($w_K$) of a component (K) ;
   d) a separate summation of all mass increments ($m(T_i)$) of the flowed substance mixture (SG) and the determined component mass increments ($m_K(T_i)$) is carried out over the filling time period ($t_0$ to $t_n$) from the mass increments ($m(T_i)$) of all sampling intervals ($T_i$ from $T_1$ to $T_n$) for the purposes of determining the mass ($m_K$) of the at least one component (K) and for the purposes of determining the overall mass ($m_{ges}$) ; and
   e) the mean content ($G_K$) of the at least one component (K) of the flowed substance mixture (SG) over the filling time period ($t_0$ to $t_n$) is determined by determining the mass fractions ($w_K$) of the at least one component (K) in the container (5) over the filling time period ($t_0$ to $t_n$) from the mass ratios of the mass ($m_K$) of the at least one component (K) to the overall mass ($m_{ges}$), wherein the mean content ($G_K$) is defined as a percentage mass fraction ($w_K$) of the at least one component (K).

2. Method according to Claim 1, **characterized in that** the measured mean content ($G_K$) of a component is made available as an actual value input variable for an automatic measuring and control system for closed-loop control of the mean content ($G_K$) of the components (K).

3. Method according to Claim 1, **characterized in that** the current content ($G_K(T_i)$) and the mass ($m_i$) of the substance mixture (SG) are measured over the sampling interval ($T_i$) using an ultrasonic flow measuring system.

4. Method according to Claim 3, **characterized in that** the calculation and integration of the component masses

($m_K(T_i)$) of the components (K) are implemented in the measuring transmitter of the ultrasonic flow measuring system and the calculation and integration of the resultant mean content ($G_K$) of the entire flowed substance mixture (SG) is provided by way of a suitable process output.

5. Method according to any one of Claims 1 to 4, **characterized in that** in the case of a plurality of feed lines (2) to a mixer (1) there is, for each feed line (2) and at each sampling time ($t_n$), a separate summation of the masses (m) of the flowed substance mixture (SG) and the component masses ($m_K$) determined therefrom, and these are assigned to a measuring channel (Knr), wherein "totalized" masses *(m)* of the individual measuring channels (Knr) are detected at the sampling time ($t_n$), summated and related to one another.

6. Method according to Claim 5, **characterized in that** a feed of one or more substance mixtures (SG) with a respective known constant content *(G)* is implemented in a separate feed line in each case.

**Revendications**

1. Procédé de détermination d'une teneur moyenne ($G_K$) en au moins un composant (K) dans un mélange de substances (SG) coulant de deux composants ou plus dans un récipient (5) à chaque instant d'échantillonnage ($t_i$) dans une période de remplissage ($t_0$ à $t_n$),

   le récipient (5) étant rempli par un afflux variable dans le temps depuis au moins une conduite d'arrivée (2) et avec une teneur momentanée variable dans le temps ($G_K(T_i)$) du composant (K),
   par

   a) détermination d'une masse ayant coulé ($m_i$) du mélange de substances (SG) au sein d'un intervalle d'échantillonnage ($T_i$) discret suffisamment petit aux instants ($t_i$) en commençant par l'instant ($t_0$) par

   a1) mesure d'un débit volumique (V) et d'une densité ($\rho$) du mélange de substances (SG) et détermination d'un débit massique (m) du mélange de substances (SG) à partir du débit volumique (V) et de la densité ($\rho$) mesurés sur un intervalle d'échantillonnage ($T_i$) et calcul d'un incrément de masse (m(Ti)) ; ou
   a2) mesure du débit massique (m) du mélange de substances (SG) sur un intervalle d'échantillonnage ($T_i$) et calcul d'un incrément de masse (m(Ti)) ;

   b) détermination de la teneur momentanée ($G_K(T_i)$) en tant que part massique ($w_K$) du mélange de substances coulant sur un intervalle d'échantillonnage ($T_i$) associé à la masse ($m_i$) du mélange de substances (SG), déterminée à l'intérieur de cet intervalle d'échantillonnage, **caractérisé en ce que**
   c) une décomposition de l'incrément de masse (m($T_i$)) du mélange de substances (SG) sur un intervalle d'échantillonnage ($T_i$) en incréments de masse de composant ($m_k(T_i)$) individuels est effectuée à partir de la masse ($m_i$) du mélange de substances (SG) et de la part massique ($w_K$) d'un composant (K) ;
   d) une addition séparée de tous les incréments de masse (m($T_i$) du mélange de substances (SG) ayant coulé et des incréments de masse de composant ($m_k(T_i)$) identifiés sur la période de remplissage ($t_0$ à $t_n$) est réalisée à partir des incréments de masse (m($T_i$) de tous les intervalles d'échantillonnage ($T_i$ de $T_1$ à $T_n$) en vue de la détermination de la masse ($m_K$) de l'au moins un composant (K) ainsi que de la détermination de la masse totale ($m_{ges}$) ;
   et
   e) la détermination de la teneur moyenne ($G_K$) en l'au moins un composant (K) dans le mélange de substances (SG) ayant coulé sur la période de remplissage ($t_0$ à $t_n$) est effectuée par la détermination de la part massique ($w_K$) de l'au moins un composant (K) dans le récipient (5) sur la période de remplissage ($t_0$ à $t_n$) à partir des rapports de masse de la masse ($m_K$) de l'au moins un composant (K) à la masse totale ($m_{ges}$), la teneur moyenne ($G_K$) étant définie comme la part massique ($w_K$) en pourcentage de l'au moins un composant (K).

2. Procédé selon la revendication 1, **caractérisé en ce que** la teneur moyenne ($G_K$) en un composant est mise à disposition comme grandeur d'entrée de valeur réelle pour un système automatique de mesure et de régulation destiné à réguler la teneur moyenne ($G_K$) en le composant (K).

3. Procédé selon la revendication 1, **caractérisé en ce que** la mesure de la teneur momentanée ($G_K(T_i)$) et de la

masse ($m_i$) du mélange de substances (SG) est effectuée sur l'intervalle d'échantillonnage ($T_i$) au moyen d'un système de mesure de débit à ultrasons.

4. Procédé selon la revendication 3, **caractérisé en ce que** le calcul et l'intégration des masses de composant ($m_k(T_i)$) du composant (K) sont effectués dans le convertisseur de mesure du système de mesure de débit à ultrasons et le décompte et l'intégration de la teneur moyenne ($G_K$) en résultant de l'ensemble du mélange de substances (SG) sont fournies par le biais d'une sortie de processus propre.

5. Procédé selon l'une des revendications 1 à 4, **caractérisé en ce qu'**en présence de plusieurs conduites d'arrivée (2) vers un mélangeur (1), une addition séparée des masses (m) du mélange de substances (SG) ayant coulé et des masses de composant ($m_K$) identifiées à partir de là est effectuée pour chaque conduite d'arrivée (2) à chaque instant d'échantillonnage ($t_n$) et sont associées à un canal de mesure (Knr), les masses (m) « totalisées » des canaux de mesure (Knr) individuels à l'instant d'échantillonnage ($t_n$) étant acquises, additionnées et mises en rapport.

6. Procédé selon la revendication 5, **caractérisé en ce qu'**une injection d'un ou plusieurs mélanges de substances (SG) est effectuée avec une teneur (G) constante respectivement connue respectivement dans une conduite d'arrivée séparée.

Figur 1

| A: $m_{HNO_3} + m_{H_2O}$ | B: $m_{H_2O}$ | Y: |
| IS+IW1 | IW2 | $G_{KS}$ |

KS 3

KW 3

6

2

2

1

4

5

7

Gehalt = Istwert

**Figur 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1725839 B1
- DE 102014115566 A1
- DE 102014106729 A1
- DE 102007062908 A1
- US 2004057334 A1
- US 2006285429 A1